# EUROPÄISCHE PATENTSCHRIFT

(11) **EP 1 023 912 B1**
(45) Veröffentlichungstag und Bekanntmachung des Hinweises auf die Patenterteilung: **23.11.2005**
(21) Anmeldenummer: 00100032.2
(22) Anmeldetag: 04.01.2000
(51) Int. Cl.: A61M 16/00, A61M 16/10

(54) **Vorrichtung zur Beatmung**
Respiratory device
Appareil respiratoire

(30) Priorität: 30.01.1999 DE 19903732
(43) Veröffentlichungstag der Anmeldung: 02.08.2000
(73) Patentinhaber: Weinmann Geräte für Medizin GmbH & Co. KG, 22525 Hamburg (DE)
(72) Erfinder: Feldhahn, Karl-Andreas, Dr., 22761 Hamburg (DE)
(74) Vertreter: Klickow, Hans-Henning

(56) Entgegenhaltungen:
- EP-A- 0 549 299
- DE-A- 4 319 458
- DE-C- 4 338 813
- US-A- 5 460 172

## Beschreibung

Die Erfindung betrifft eine Vorrichtung zur Beatmung, die eine Atemgaspumpe, einen Verbindungsschlauch, eine Beatmungsmaske sowie ein Ausatmungselement aufweist und bei der im Bereich eines die Atemgaspumpe halternden Gerätegehäuses ein Gerätefilter zur Zurückhaltung von Luftverunreinigungen angeordnet ist, sowie bei der die Atemgaspumpe eine Steuerung derart aufweist, daß sowohl innerhalb von Einatmungsphasen als auch innerhalb von Ausatmungsphasen ein nasaler kontinuierlicher positiver Atemwegsdruck bereitgestellt ist.

Derartige vorrichtungen werden typischerweise eingesetzt, wenn bei Patienten schlafbezogene Atmungsstörungen behandelt werden müssen.

Der grundsätzliche Aufbau der erforderlichen Vorrichtungen sowie eine Erläuterung der bekannten Therapieverfahren werden im Buch "Schlafapnoe und Heimbeatmungen, Holger Hein und Detlef Kirsten, 2. Auflage 1997, Dustri-Verlag Dr. Karl Feistle beschrieben.

Ein grundsätzliches Problem bei der Durchführung von Dauerbeatmungen besteht darin, daß dem Körper des Patienten Wärme und Feuchtigkeit entzogen werden. Typischerweise wird deshalb in gerätetechnischer Hinsicht zusätzlich zum eigentlichen Beatmungsgerät ein separater Luftbefeuchter und Lufterwärmer verwendet. Bekannt ist beispielsweise die Verwendung von Kaltanfeuchtern, bei denen die für die Atmung vorgesehene Luft über eine Wasseroberfläche streicht und sich dabei mit Feuchtigkeit anreichert. Die Atemluft wird jedoch nicht angewärmt und das Ausmaß der erreichbaren Feuchtigkeitsanreicherung ist begrenzt.

Darüber hinaus werden Warmanfeuchter eingesetzt, die geregelt und beheizt werden können, um einen höheren Anfeuchtegrad zu gewährleisten. In Abhängigkeit von einem jeweiligen Umgebungsklima ist es jedoch möglich, daß hierdurch eine 100%tige Sättigung der Atemluft mit Feuchtigkeit erreicht wird und daß hierdurch im Bereich des Verbindungsschlauches eine Kondensation erfolgt. Ebenfalls kann nicht ausgeschlossen werden, daß eine dauerhafte Überfeuchtung der Atemwege verursacht wird, die eine Funktion der Flimmerhärchen beeinträchtigt oder sogar blockiert.

Generell sind sowohl die Kaltanfeuchter als auch die Warmanfeuchter technisch relativ aufwendig sowie umständlich in ihrer Handhabung und bezüglich ihrer Reinigung.

In der DE-A 43 19 458 werden sogenannte HME-Filter beschrieben, die bei Notfallbeatmungen dazu verwendet werden, vom Patienten ausgeatmete Feuchtigkeit und Wärme zurückzuhalten und einen Durchlaß von Krankheitskeimen und anderen Mikroorganismen zu verhindern. Die bekannten derartigen Filter weisen jedoch den Nachteil auf, daß bei einer Gasdurchströmung ein relativ hoher Druckabfall auftritt, der zu einem materialtechnisch in einem größeren Streuungsbereich liegt und der darüber hinaus in Abhängigkeit von den jeweiligen Einsatzbedingungen variiert. Eine unmittelbare Verwendung dieser Filter im Bereich einer Dauerbeatmung von schlafbezogenen Atmungsstörungen unter Bereitstellung eines kontinuierlichen positiven Atemwegsdruckes ist deshalb nicht möglich.

In der EP-A 0 549 299 wird eine Vorrichtung zur Beatmung beschrieben, bei der im Zusammenhang mit der Anwendung der CPAP-Therapie im Bereich der Luftzuführung einer Beatmungsmaske ein Filter sowie ein Befeuchter angeordnet sind.

In der DE-C 43 38 813 wird eine Vorrichtung zur Verwendung im Bereich der CPAP-Therapie beschrieben, wobei die Vorrichtung mit einem beheizbaren und regelbaren Anfeuchter versehen ist.

Aus der US-A-5,460,172 ist ein HME-Filter für Anwendungen im Zusammenhang mit der Tracheotonomie bekannt. Die geometrische Gestaltung des HME ist an den vorgesehenen Anwendungsfall angepaßt.

Aufgabe der vorliegenden Erfindung ist es, eine Vorrichtung der einleitend genannten Art derart zu konstruieren, daß verbesserte Beatmungseigenschaften bereitgestellt werden.

Diese Aufgabe wird erfindungsgemäß dadurch gelöst, daß im Bereich der Beatmungsmaske und des Ausatmungselementes mindestens ein Filter zur Zurückhaltung von Wärme und Feuchtigkeit angeordnet ist, daß der Filter einen Strömungswiderstand von weniger als 20 mbar bei einer Strömung von 60 l/min aufweist und daß das Filtermaterial innerhalb des Filters austauschbar angeordnet ist.

Weitere Aufgabe der vorliegenden Erfindung ist es, ein Verfahren der einleitend genannten Art derart anzugeben, daß die physiologische Qualität des Atemgases verbessert wird.

Diese Aufgabe wird erfindungsgemäß dadurch gelöst, daß in örtlicher Nähe zur Beatmungsmaske aus der Beatmungsmaske herausströmendes Atemgas derart gefiltert wird, daß Anteile an Feuchtigkeit und Wärme zurückgehalten werden und daß die Filterung derart durchgeführt wird, daß am Filter zur Zurückhaltung von Feuchtigkeit und Wärme eine Druckdifferenz von weniger als 20 mbar bei einer Strömung von 60 l/min erzeugt wird.

Durch den gewählten Montageort für den Filter zur Zurückhaltung von Wärme und Feuchtigkeit sowie die Filterdimensionierung kann erreicht werden, daß Wärmeverluste und Feuchtigkeitsverluste weitgehend vermieden werden. Es ist insbesondere möglich, ein äußerst kompakt ausgebildetes Beatmungsgerät bereitzustellen, das vom jeweiligen Patienten auch auf Reisen mitgenommen werden kann. Ein Betrieb des Gerätes ist bei einer großen Vielfalt von Umgebungsbedingungen möglich, ohne daß die Qualität des Atemgases beeinträchtigt wird. Insbesondere ist es auch möglich, den gewünschten kontinuierlichen positiven Atemwegsdruck unabhängig von den konkreten Umweltbedingungen in einem engen Toleranzintervall vorzugeben.

Die erfindungsgemäße Aufbereitung des Atemgases unterstützt ebenfalls sowohl eine mobile Anwendbarkeit als auch geringe Gerätepreise sowie geringe Betriebskosten.

Eine eng tolerierte Aufrechterhaltung des nasalen kontinuierlichen positiven Atemwegsdruckes wird insbesondere dadurch unterstützt, daß der Strömungswiderstand weniger als 2 mbar bei einer Strömung von 60 l/min beträgt.

Ein kompakter Aufbau wird dadurch unterstützt, daß der Filter als Teil der Beatmungsmaske ausgebildet ist.

Eine Verwendung standardisierter Bauelemente wird dadurch unterstützt, daß der Filter zwischen der Beatmungsmaske und dem Ausatmungselement angeordnet ist.

Gemäß einer weiteren Ausführungsform ist es auch möglich, daß der Filter als Teil des Ausatmungselementes ausgebildet ist.

Insbesondere ist daran gedacht, daß der Filter im Bereich einer der Beatmungsmaske zugewandten Begrenzung eines Schalldämpfers des Ausatmungselementes angeordnet ist.

Zur Bereitstellung genauer Meßwerte wird vorgeschlagen, daß durch den Filter ein Druckmeßschlauch hindurchgeschleift ist.

Zur Verringerung der Betriebskosten wird vorgeschlagen, daß das Filtermaterial innerhalb des Filters austauschbar angeordnet ist.

Eine mögliche Materialwahl besteht darin, daß das Filtermaterial aus einer Kunststoffwatte ausgebildet ist.

Zur Verbesserung der Eigenschaften hinsichtlich der Speicherung von Feuchtigkeit wird vorgeschlagen, daß das Filtermaterial Salzanteile enthält.

Insbesondere ist daran gedacht, daß die Salzanteile hygroskopisch ausgebildet sind.

Die Zurückhaltungswirkung kann ebenfalls dadurch unterstütz werden, daß das Filtermaterial Elektrete zur Erzeugung eines permanenten elektrischen Feldes enthält.

Eine zweckmäßige Materialwahl besteht darin, daß das Filtermaterial mindestens bereichsweise aus Polypropylen ausgebildet ist.

Ebenfalls ist daran gedacht, daß das Filtermaterial mindestens bereichsweise aus Polyurethan ausgebildet ist.

Eine weitere Möglichkeit zur Realisierung der Filtereigenschaften besteht darin, daß im Bereich des Filters mindestens eine Membran angeordnet ist.

Ebenfalls ist es möglich, daß das Filtermaterial aus einer geschäumten Substanz ausgebildet ist.

Eine weitere Materialauswahl besteht darin, daß das Filtermaterial aus einem faserartigen Material ausgebildet ist.

Eine besonders leichte und einfach auswechselbare Konstruktion wird dadurch bereitgestellt, daß das Filtermaterial mindestens bereichsweise aus Papier ausgebildet ist.

Ein kompakter Aufbau wird auch dadurch unterstützt, daß der Filter im Bereich des Verbindungsschlauches angeordnet ist.

Eine weitere Variante zur Unterstützung einer Druckmessung besteht darin, daß durch den Filter eine Signalleitungseinrichtung hindurchgeschleift ist. Alternativ zu der bereits erwähnten Verwendung eines Druckmeßschlauches ist es auch denkbar, lokal einen Drucksensor anzuordnen und die entsprechenden Signale elektrisch weiterzuleiten. Bei einer derartigen Ausführungsform würde ein Meßkabel durchgeschleift werden. In Abhängigkeit von der jeweiligen Anwendung kann auf ein Durchschleifen aber auch verzichtet werden.

In den Zeichnungen sind Ausführungsbeispiele der Erfindung schematisch dargestellt. Es zeigen:
- Fig.1: eine perspektivische Darstellung zur Veranschaulichung des prinzipiellen Aufbaues einer Vorrichtung zur Beatmung,
- Fig.2: eine vergrößerte Darstellung der Vorrichtung im Bereich der Beatmungsmaske und des Ausatmungselementes, wobei der Filter im Bereich der Atemmaske angeordnet ist,

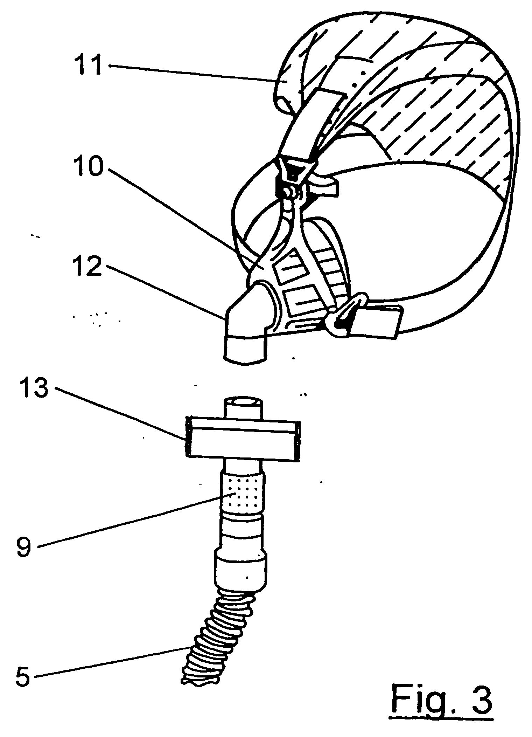

- Fig.3: eine Darstellung entsprechend Fig. 2 bei einer Anordnung des Filters im Bereich des Ausatmungselementes
**und**
- Fig.4: einen vergrößerten Querschnitt durch einen Filter zur Zurückhaltung von Wärme und Feuchtigkeit mit durchgeschleifter Meßleitung zur Druckerfassung.

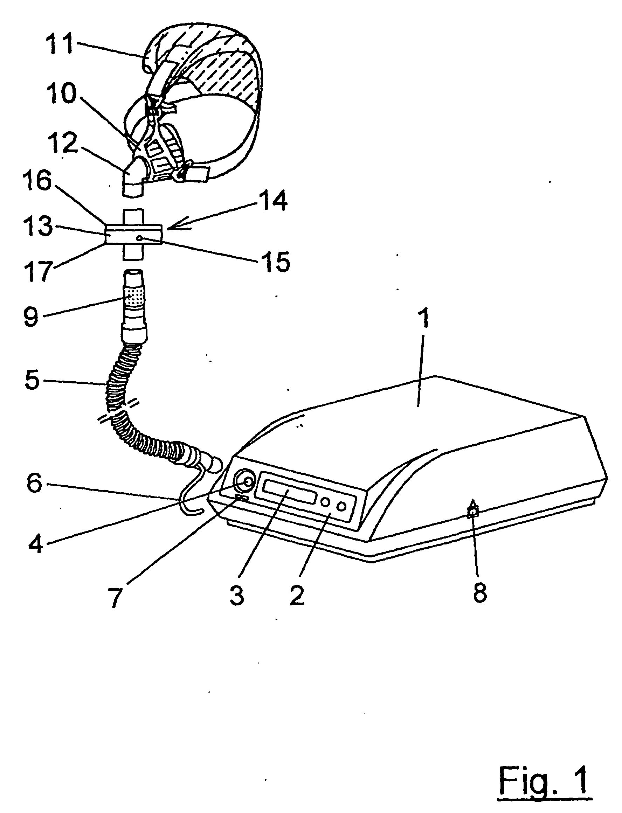

Fig. 1 zeigt den grundsätzlichen Aufbau einer Vorrichtung zur Beatmung bei gleichzeitiger Erzeugung eines nasalen kontinuierlichen positiven Atemwegsdruckes. Im Bereich eines Gerätegehäuses (1) mit Bedienfeld (2) sowie Anzeige (3) ist in einem Geräteinnenraum eine Atemgaspumpe angeordnet. Über eine Kopplung (4) wird ein Verbindungsschlauch (5) angeschlossen. Entlang des Verbindungsschlauches (5) verläuft ein zusätzlicher Druckmeßschlauch (6), der über einen Druckeingangsstutzen (7) mit dem Gerätegehäuse (1) verbindbar ist. Zur Ermöglichung einer Datenübertragung weist das Gerätegehäuse (1) eine Schnittstelle (8) auf.

Im Bereich einer dem Gerätegehäuse (1) abgewandten Ausdehnung des Verbindungsschlauches (5) ist ein Ausatmungselement (9) angeordnet. Das Ausatmungselement (9) kann beispielsweise aus einem porösen, faserartigen oder schaumartigen Material ausgebildet sein.

Fig. 1 zeigt darüber hinaus eine Beatmungsmaske (10), die als Nasalmaske ausgebildet ist. Eine Fixierung im Bereich eines Kopfes eines Patienten kann über eine Kopfhaube (11) erfolgen. Im Bereich ihrer dem Verbindungsschlauch (5) zugewandten Ausdehnung weist die Beatmungsmaske (10) einen Anschlußstutzen (12) auf.

Bei der in Fig. 1 dargestellten Ausführungsform ist zwischen dem Verbindungsschlauch (5) und der Beatmungsmaske (10) ein Filter (13) zur Zurückhaltung von Wärme und Feuchtigkeit angeordnet. Der Filter (13) weist ein Filtergehäuse (14) auf, in dem Filtermaterial (15) angeordnet ist. Zur Ermöglichung einer Austauschbarkeit des Filtermaterials (15) ist es möglich, das Filtergehäuse (14) aus einem Gehäuseoberteil (16) und einem Gehäuseunterteil (17) auszubilden, die reversibel miteinander verbunden und voneinander getrennt werden können.

Als Filtermaterialien können beispielsweise Fasern oder offenporige Schäume aus Polypropylen oder Polyurethan verwendet werden. Ebenfalls ist es möglich, geeignete Membranen zu verwenden. Eine Feuchtigkeitsrückhaltung kann durch die Verwendung hygroskopischer Salze verbessert werden. Ebenfalls ist es möglich, Elektrete einzusetzen, die materialbedingt ein permanentes elektrisches Feld bereitstellen.

Durch die Verwendung des Filters (13) wird verhindert, daß ausgeatmete Feuchtigkeit sowie mit dem Atemgas abgeführte Wärme bis in den Bereich des Ausatmungselementes (9) und durch dieses hindurch in eine Umgebung gelangen. Die Wärme und die Feuchtigkeit werden vielmehr im Bereich des Filters (13) bei einem Ausatmungsvorgang angelagert und strömen bei einem Einatmungsvorgang durch die Beatmungsmaske (10) hindurch zum Patienten zurück.

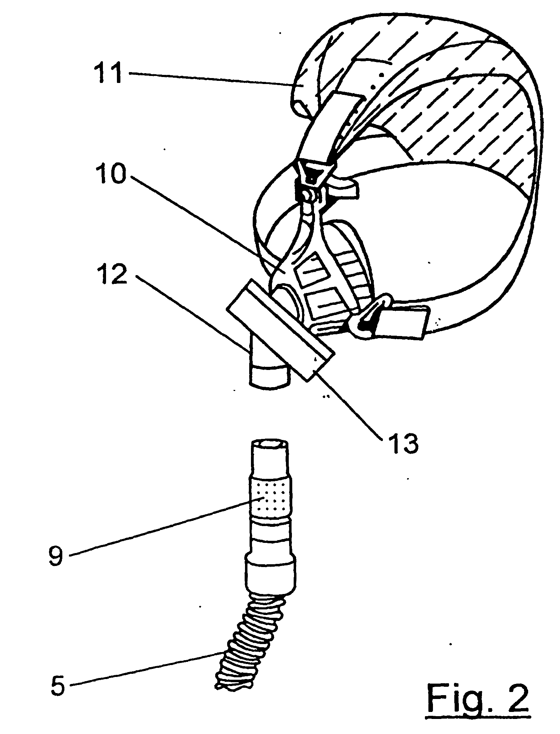

Fig. 2 zeigt eine Ausführungsform, bei der der Filter (13) als Teil der Beatmungsmaske (10) ausgebildet ist. Durch eine derartige Ausführungsform kann eine kompakte Gestaltung bei Verringerung der Teileanzahl erreicht werden.

Eine weitere Variante zur Realisierung ist zu Fig. 13 dargestellt. Hier ist der Filter (13) als Teil des Ausatmungselementes (9) realisiert. Auch hierdurch kann eine kompakte Ausführungsform bei geringer Teileanzahl erreicht werden.

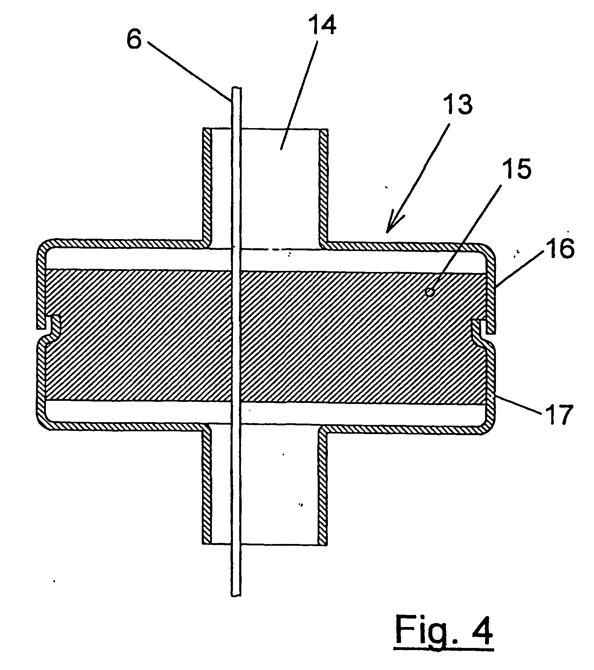

Fig. 4 zeigt in einem Querschnitt einen möglichen Aufbau für den Filter (13). Es ist insbesondere erkennbar, daß durch den Filter (13) und durch das Filtermaterial (15) hindurch der Druckmeßschlauch (6) hindurchgeschleift ist. Hierdurch wird es ermöglicht, unmittelbar im Bereich der Beatmungsmaske (10) einen aktuellen Druck zu erfassen und dem Gerätegehäuse (10) mit den dort vorgesehenen Steuerelementen zuzuführen. Hierdurch wird die Aufrechterhaltung des nasalen kontinuierlichen positiven Atemwegsdrucks innerhalb eines geringen Toleranzintervalls unterstützt. Grundsätzlich ist es bei einer geeigneten Dimensionierung der Filters (13) hinsichtlich des Strömungswiderstandes aber auch möglich, die Druckmessung im Bereich des Gerätegehäuses (1), beziehungsweise zwischen dem Filter (13) und der Atemgaspumpe, durchzuführen. Eine derartige Messung ist insbesondere zweckmäßig, wenn der Strömungswiderstand des Filters (13) bei einer Strömung von 60 l/min höchstens 2 mbar beträgt.

Im Bereich des Gerätegehäuses (10) sind zusätzlich ein Grobfilter zur Zurückhaltung von Verunreinigungen und Keimen sowie die erforderlichen elektrischen und elektronischen Bauelemente angeordnet.

## Patentansprüche

1. Vorrichtung zur Beatmung, die eine Atemgaspumpe, einen Verbindungsschlauch (5), eine Beatmungsmaske (10) sowie ein Ausatmungselement (9) aufweist und bei der die Atemgaspumpe eine Steuerung derart aufweist, daß sowohl innerhalb von Einatmungsphasen als auch innerhalb von Ausatmungsphasen ein nasaler kontinuierlicher positiver Atemwegsdruck, bereitgestellt ist, wobei im Bereich der Beatmungsmaske (10) und des Ausatmungselementes (9) mindestens ein Filter (13) zur Zurückhaltung von wärme und Feuchtigkeit angeordnet ist, **dadurch gekennzeichnet, daß** im Bereich eines die Atemgaspumpe halternden Gerätegehäuses (1) ein Gerätefilter zur Zurückhaltung von Luftverunreinigungen angeordnet ist und, daß der Filter (13) einen Strömungswiderstand von weniger als 20 mbar bei einer Strömung von 60 l/min aufweist und daß das Filtermaterial (15) innerhalb des Filters (13) austauschbar angeordnet ist.

2. Vorrichtung nach Anspruch 1, **dadurch gekennzeichnet, daß** der Strömungswiderstand weniger als 2 mbar bei einer Strömung von 60 l/min beträgt.

3. Vorrichtung nach Anspruch 1 oder 2, **dadurch gekennzeichnet, daß** der Filter (13) als Teil der Beatmungsmaske (10) ausgebildet ist.

4. Vorrichtung nach Anspruch 1 oder 2, **dadurch gekennzeichnet, daß** der Filter (13) zwischen der Beatmungsmaske (10) und dem Ausatmungselement (9) angeordnet ist.

5. Vorrichtung nach Anspruch 1 oder 2, **dadurch gekennzeichnet, daß** der Filter (13) als Teil des Ausatmungselementes (9) ausgebildet ist.

6. Vorrichtung nach Anspruch 5, **dadurch gekennzeichnet, daß** der Filter (13) im Bereich einer der Beatmungsmaske (10) zugewandten Begrenzung eines Schalldämpfers des Ausatmungselementes (9) angeordnet ist.

7. Vorrichtung nach einem der Ansprüche 1 bis 6, **dadurch gekennzeichnet, daß** durch den Filter (13) ein Druckmeßschlauch (6) hindurchgeschleift ist.

8. Vorrichtung nach einem der Ansprüche 1 bis 7, **dadurch gekennzeichnet, daß** das Filtermaterial (15) aus einer Kunststoffwatte ausgebildet ist.

9. Vorrichtung nach einem der Ansprüche 1 bis 8, **dadurch gekennzeichnet, daß** das Filtermaterial (15) Salzanteile enthält.

10. Vorrichtung nach Anspruch 9, **dadurch gekennzeichnet, daß** die Salzanteile hygroskopisch ausgebildet sind.

11. Vorrichtung nach einem der Ansprüche 1 bis 10, **dadurch gekennzeichnet, daß** das Filtermaterial (15) Elektrete zur Erzeugung eines permanenten elektrischen Feldes enthält.

12. Vorrichtung nach einem der Ansprüche 1 bis 11, **dadurch gekennzeichnet, daß** das Filtermaterial (15) mindestens bereichsweise aus Polypropylen ausgebildet ist.

13. Vorrichtung nach einem der Ansprüche 1 bis 12, **dadurch gekennzeichnet, daß** das Filtermaterial (15) mindestens bereichsweise aus Polyurethan ausgebildet ist.

14. Vorrichtung nach einem der Ansprüche 1 bis 13, **dadurch gekennzeichnet, daß** im Bereich des Filters (13) mindestens eine Membran angeordnet ist.

15. Vorrichtung nach einem der Ansprüche 1 bis 14, **dadurch gekennzeichnet, daß** das Filtermaterial (15) aus einer geschäumten Substanz ausgebildet ist.

16. Vorrichtung nach einem der Ansprüche 1 bis 15, **dadurch gekennzeichnet, daß** das Filtermaterial (15) aus einem faserartigen Material ausgebildet ist.

17. Vorrichtung nach einem der Ansprüche 1 bis 16, **dadurch gekennzeichnet, daß** das Filtermaterial (15) mindestens bereichsweise aus Papier ausgebildet ist.

18. Vorrichtung nach einem der Ansprüche 1 bis 17, **dadurch gekennzeichnet, daß** der Filter (13) im Bereich des Verbindungsschlauches (5) angeordnet ist.

19. Vorrichtung nach einem der Ansprüche 1 bis 18, **dadurch gekennzeichnet, daß** durch den Filter (13) eine Signalleitungseinrichtung hindurchgeschleift ist.

## Claims

1. A device for breathing, which has a breathing gas pump, a connection hose (5), a breathing mask (10) and an exhaling element (9), and in which the breathing gas pump has a control such that a continuous positive nasal pressure in the airways is provided both during inhaling phases and during exhaling phases, in which at least one filter (13) for retaining heat and moisture is arranged in the region of the breathing mask (10) and the exhaling element (9), **characterised in that** a device filter is arranged in the region of a device housing (1) holding the breathing gas pump in order to retain impurities in the air, and **in that** the filter (13) has a flow resistance of less than 20 mbar at a flow rate of 60 l/min, and **in that** the filter material (15) is arranged replaceably within the filter (13).

2. A device according to Claim 1, **characterised in that** the flow resistance is less than 2 mbar at a flow rate of 60 l/min.

3. A device according to Claim 1 or 2, **characterised in that** the filter (13) is constructed as part of the breathing mask (10).

4. A device according to Claim 1 or 2, **characterised in that** the filter (13) is arranged between the breathing mask (10) and the exhaling element (9).

5. A device according to Claim 1 or 2, **characterised in that** the filter (13) is constructed as part of the exhaling element (9).

6. A device according to Claim 5, **characterised in that** the filter (13) is arranged in the region of a delimitation, facing the breathing mask (10), of a soundproofing means of the exhaling element (9).

7. A device according to one of Claims 1 to 6, **characterised in that** a pressure-measuring hose (6) is looped through the filter (13).

8. A device according to one of Claims 1 to 7, **characterised in that** the filter material (15) is made from a synthetic material similar to cotton wool.

9. A device according to one of Claims 1 to 8, **characterised in that** the filter material (15) contains salt portions.

10. A device according to Claim 9, **characterised in that** the salt portions are in hygroscopic form.

11. A device according to one of Claims 1 to 10, **characterised in that** the filter material (15) contains electrets in order to generate a permanent electrical field.

12. A device according to one of Claims 1 to 11, **characterised in that** the filter material (15) is made at least in certain regions from polypropylene.

13. A device according to one of Claims 1 to 12, **characterised in that** the filter material (15) is made at least in certain regions from polyurethane.

14. A device according to one of Claims 1 to 13, **characterised in that** at least one membrane is arranged in the region of the filter (13).

15. A device according to one of Claims 1 to 14, **characterised in that** the filter material (15) is made from a foamed substance.

16. A device according to one of Claims 1 to 15, **characterised in that** the filter material (15) is made from a fibrous material.

17. A device according to one of Claims 1 to 16, **characterised in that** the filter material (15) is made at least in certain regions from paper.

18. A device according to one of Claims 1 to 17, **characterised in that** the filter (13) is arranged in the region of the connection hose (5).

19. A device according to one of Claims 1 to 18, **characterised in that** a signal-feeding device is looped through the filter (13).

## Revendications

1. Appareil d'assistance respiratoire, qui comprend une pompe à gaz respiratoire, un tuyau flexible de liaison (5), un masque respiratoire (10), de même qu'un organe d'expiration (9), et dans lequel la pompe à gaz respiratoire est commandée de telle sorte que, aussi bien au cours des phases d'inspiration qu'également au cours des phases d'expiration, une pression de circuit respiratoire positive continue est assurée au niveau du nez, au moins un filtre (13) étant disposé dans la région du masque respiratoire (10) et de l'organe d'expiration (9), pour retenir la chaleur et l'humidité, **caractérisé en ce qu'**un filtre d'appareillage pour retenir des impuretés de l'air est agencé dans la région d'un boîtier (1) d'appareillage contenant la pompe à gaz respiratoire, et **en ce que** le filtre (13) présente une résistance de passage inférieure à 20 mbar pour un flux de 60 l/min, et **en ce que** le matériau filtrant (15) à l'intérieur du filtre (13) peut être changé.

2. Appareil selon la revendication 1, **caractérisé en ce que** la résistance de passage est inférieure à 2 mbar pour un flux de 60 l/min.

3. Appareil selon la revendication 1 ou 2, **caractérisé en ce que** le filtre (13) est réalisé comme une partie du masque respiratoire (10).

4. Appareil selon la revendication 1 ou 2, **caractérisé en ce que** le filtre (13) est agencé entre le masque respiratoire (10) et l'organe d'expiration (9).

5. Appareil selon la revendication 1 ou 2, **caractérisé en ce que** le filtre (13) est réalisé comme une partie de l'organe d'expiration (9).

6. Appareil selon la revendication 5, **caractérisé en ce que** le filtre (13) est disposé dans la région d'une limite d'un élément d'insonorisation de l'organe d'expiration (9), tournée vers le masque respiratoire (10).

7. Appareil selon l'une des revendications 1 à 6, **caractérisé en ce qu'**un tuyau flexible (6) de mesure de la pression traverse le filtre (13).

8. Appareil selon l'une des revendications 1 à 7, **caractérisé en ce que** le matériau filtrant (15) est réalisé en ouate synthétique.

9. Appareil selon l'une des revendications 1 à 8, **caractérisé en ce que** le matériau filtrant (15) contient une proportion de sel.

10. Appareil selon la revendication 9, **caractérisé en ce que** la proportion de sel est hygroscopique.

11. Appareil selon l'une des revendications 1 à 10, **caractérisé en ce que** le matériau filtrant (15) contient des électrets pour générer un champs électrique permanent.

12. Appareil selon l'une des revendications 1 à 11, **caractérisé en ce que** le matériau filtrant (15) est réalisé au moins en partie en polypropylène.

13. Appareil selon l'une des revendications 1 à 12, **caractérisé en ce que** le matériau filtrant (15) est réalisé au moins en partie en polyuréthanne.

14. Appareil selon l'une des revendications 1 à 13, **caractérisé en ce qu'**au moins une membrane est prévue dans la région du filtre (13).

15. Appareil selon l'une des revendications 1 à 14, **caractérisé en ce que** le matériau filtrant (15) est réalisé à partir d'une substance en mousse.

16. Appareil selon l'une des revendications 1 à 15, **caractérisé en ce que** le matériau filtrant (15) est réalisé à partir d'une matière fibreuse.

17. Appareil selon l'une des revendications 1 à 16, **caractérisé en ce que** le matériau filtrant (15) est réalisé au moins en partie en papier.

18. Appareil selon l'une des revendications 1 à 17, **caractérisé en ce que** le filtre (13) est disposé dans la région du tuyau flexible (5) de liaison.

19. Appareil selon l'une des revendications 1 à 18, **caractérisé en ce qu'**un dispositif conducteur de signal traverse le filtre (13).
